# EUROPÄISCHE PATENTANMELDUNG

(11) **EP 4 717 196 A1**
(43) Veröffentlichungstag der Anmeldung: **01.04.2026**
(21) Anmeldenummer: 24203275.3
(22) Anmeldetag: 27.09.2024
(51) Int. Cl.: A61B 17/30, A61B 34/20, A61B 90/00, A61B 17/34, A61B 6/50, A61B 10/02, A61B 17/00

(54) **VERFAHREN UND VORRICHTUNG ZUR AUTOMATISIERTEN BESTIMMUNG DER POSITION EINER NADELSPITZE EINER BIOPSIENADEL**

(71) Anmelder: Siemens Healthineers AG, 91301 Forchheim (DE)
(72) Erfinder: Zimmermann, Ulf, 09623 Frauenstein / OT Nassau (DE); Gollwitzer, Helmut, 92681 Erbendorf (DE); Newrzella, Sebastian, 95447 Bayreuth (DE); Schliermann, Claus-Günter, 95478 Kemnath (DE); Mahmeen, Mohd, 560068 Bangalore (IN); Bolten, Josef, 53229 Bonn (DE); Gkekas, Konstantinos, 95448 Bayreuth (DE); Ratzmer, Karin, 91161 Hilpoltstein-Meckenhausen (DE)
(74) Vertreter: Siemens Healthineers Patent Attorneys

(57) **Zusammenfassung**

Die Erfindung betrifft ein Verfahren zur automatisierten Bestimmung der Position einer Nadelspitze (N) einer Biopsienadel (32), welche in einem Nadelhalter (31) angebracht ist und für eine Biopsie durch eine Biopsie-Einheit (30) auf ihrer longitudinalen Achse verschiebbar ist und mittels einer Nadelführung (33) geführt wird, das Verfahren umfassend die Schritte:
- Positionieren der Biopsienadel (32) im Nadelhalter (31),
- Ermitteln zumindest der Position der Nadelspitze (N) der Biopsienadel (32) mittels eines Sensors (35),
- automatisches Berechnen eines Abstandes von der Nadelspitze (N) zu einem Bezugspunkt. Des Weiteren umfasst die Erfindung eine Vorrichtung und ein Mammographiesystem.

## Beschreibung

Die Erfindung betrifft ein Verfahren und eine Vorrichtung zur automatisierten Bestimmung der Position einer Nadelspitze einer Biopsienadel sowie ein Mammographiesystem.

Bei Anwendung einer Biopsie im Rahmen der Diagnostik z.B. in der Mammographie kommen Stanz- oder Vakuumnadeln zum Einsatz, deren Länge etwa 90 mm bis 150 mm betragen kann. Die Nadelparameter werden oftmals in einer verwendeten Workstation hinterlegt und mittels dieser Nadelparameter die Zielposition einer motorisch verfahrbaren Biopsie-Einheit berechnet und angefahren. Dazu wird die gewünschte Biopsienadel in einen Nadelhalter (auch als "Gun" bezeichnet) eingesetzt, welcher dann durch einen Schlitten der Biopsie-Einheit, welcher üblicherweise manuell bewegt wird, verschoben werden kann. Durch Verschiebung des Nadelhalters auf dem Schlitten wird dann die Biopsienadel in einen Patienten geschoben. Hat die Spitze der Biopsienadel den gewünschten Punkt erreicht, wird ein Unterdruck in ihr erzeugt und dadurch eine Gewebeprobe in die Biopsienadel (oder im Folgenden auch kurz "Nadel") hereingezogen. Die Ermittlung und Berechnung der Koordinaten des gewünschten Punktes wird basierend auf mehreren Aufnahmen typischerweise mittels "Stereo"-Berechnung oder Rekonstruktion eines 3D-Datensatzes durchgeführt.

Nachdem eine Vorpositionierung des Nadelhalters durchgeführt wurde, wird die Nadel in der Praxis typischerweise in das Untersuchungsobjekt z.B. mittels eines linearen Vorschubs des Nadelhalters in den Körper eingeführt. Die Nadel wird dabei von einer sterilen Nadelführung geführt.

Die Position der Nadel im Körper wird mittels wiederholter Röntgenaufnahmen ermittelt. Diese bringen jedoch jedes Mal eine Strahlungsdosis in das Gewebe ein, die vermeidbar wäre, wenn die Position der Nadel sicher bekannt wäre. Außerdem muss darauf geachtet werden, dass der gesamte Aufbau der Biopsie-Einheit keinen Schatten in den Aufnahmen wirft. Daher wird die Nadel bevorzugt schräg angeordnet.

Vor dem gewünschten Punkt, z.B. einer Läsion, wird die Nadel meist gestoppt um z.B. mittels Röntgenaufnahmen kontrollieren zu können, ob die Nadel korrekt positioniert wurde. Wird der Nadelkörper bis zum Endanschlag eingeführt, dann ist davon auszugehen, dass die gewünschte vorausberechnete Position im Objekt (hier noch vor der Läsion) erreicht wurde. Im Anschluss wird über Federmechanismen, mechanische Antriebe oder per Vakuum die Nadel in die Endposition zur Gewebeentnahme eingeführt und die Gewebeproben entnommen.

Durch eine falsche Führung der Nadel oder die Verwendung eines falschen Nadeltyps können schwerwiegende Probleme auftreten.

Da Nadelhersteller in der Regel mehrere Nadellängen anbieten, damit Gewebeproben in unterschiedlichen Tiefen des Untersuchungsobjektes entnommen werden können, besteht die Gefahr, bei Einsatz einer falschen Nadel das beabsichtigte Gewebe zu verfehlen bzw. das Objekt komplett zu durchstoßen und damit Verletzungen des Patienten und Schäden am Untersuchungsgerät zu verursachen. Wurde dem System z.B. die falsche Nadellänge mitgeteilt, kann dies zu (ggf. gravierenden) Verletzungen führen.

Die Ermittlung der Nadellänge erfolgt nur durch manuelle Messung oder Entnahme der Länge aus Produktdaten oder Kundendokumentation. Derzeit wird die Anwesenheit einer Biopsienadel über die Anwesenheit des Nadelhalters detektiert bzw. z.B. Schalthebel umgelegt um die Nadel einführen zu können, wobei der Hebel mit einem Schalter verbunden ist, der Gerätebewegungen und Bewegungen der Nadel sperrt.

Zusammenfassend kann gesagt werden, dass die Hauptgefahr eine Fehlpositionierung der Nadelspitze im Körper darstellt. Diese Fehlpositionierung kann dadurch erfolgen, dass die Nadel falsch in den Körper eingeführt wird oder dass Nadellänge und mitgeteilte Information über die Nadellänge nicht übereinstimmen (falsche Nadel eingesetzt oder falsche Angaben zur Nadellänge gemacht). Eine Fehlpositionierung kann durch Röntgenaufnahmen erkannt werden. Diese belasten jedoch den Körper des Patienten mit einer Röntgendosis und eine Korrektur muss manuell erfolgen. Eine falsche Positionierung kann durchaus übersehen werden.

Es ist eine Aufgabe der vorliegenden Erfindung, ein Verfahren und eine Vorrichtung zur automatisierten Bestimmung der Position einer Nadelspitze einer Biopsienadel sowie ein Mammographiesystem anzugeben, mit denen die oben beschriebenen Nachteile vermieden werden.

Diese Aufgabe wird durch ein Verfahren gemäß Patentanspruch 1, eine Vorrichtung gemäß Patentanspruch 10 und ein Mammographiesystem gemäß Patentanspruch 13 gelöst.

Ein erfindungsgemäßes Verfahren dient zur automatisierten Bestimmung der Position einer Nadelspitze einer Biopsienadel, welche in einem Nadelhalter angebracht ist und für eine Biopsie durch eine Biopsie-Einheit. auf ihrer longitudinalen Achse verschiebbar ist und mittels einer Nadelführung geführt wird. Das Verfahren umfasst die folgenden Schritte:
- Positionieren der Biopsienadel im Nadelhalter,
- Ermitteln zumindest der Position der Nadelspitze der Biopsienadel mittels eines Sensors,
- automatisches Berechnen eines Abstandes von der Nadelspitze zu einem Bezugspunkt.

Informationen über diesen Abstand werden dann als Information an einen Bediener und/oder in Form von Steuerbefehlen ausgegeben. Beispielsweise kann eine Warnung ausgegeben werden, dass eine falsche Nadel eingesetzt worden ist, oder ein Vorschub eines Nadelhalters blockiert werden.

Für das Verfahren ist die Biopsienadel in einem Nadelhalter angebracht. Dieser Nadelhalter ist für eine Biopsie durch eine Biopsie-Einheit (insbesondere auf einem Schiebeschlitten) auf ihrer longitudinalen Achse verschiebbar. Die Nadel wird bei einer Verschiebung mittels einer (sterilen) Nadelführung geführt. Direkt nach dem Einsetzen liegt die Nadel typischerweise noch nicht in der Nadelführung. Erst wenn der Nadelhalter etwas verschoben wurde, bewegt sich die Nadelspitze durch die Nadelführung und wird dann auf ihrem Weg durch die Nadelführung geführt. Dies alles ist im Stand der Technik gut bekannt.

Was die Verfahrensschritte betrifft, ist der dort bestimmte Abstand sowohl für die Ermittlung der Nadellänge als auch für die Ermittlung der Nadelposition verwendbar. Bei der Ermittlung sowohl der Länge der Biopsienadel als auch zur Ermittlung der Position der Biopsienadel müssen die Position der Nadelspitze und die Position eines Bezugspunktes (z.B. dem Nadelhalter oder der Nadelführung) bekannt sein. Eine Position des Nadelhalters muss nicht unbedingt gemessen werden, da sie in dessen Anfangsstellung auch aufgrund des Aufbaus der Biopsie-Einheit schon vorab bekannt sein kann. Die Bestimmung der Position der Nadelspitze der Biopsienadel kann durchaus zusätzlich durch Röntgenaufnahmen überwacht werden, dies ist jedoch nicht unbedingt notwendig.

Es wird nun die Biopsienadel auf bekannte Weise zunächst im Nadelhalter positioniert.

Als nächstes wird zumindest die Position der Nadelspitze der Biopsienadel mittels eines Sensors ermittelt. Bevorzugt wird auch noch die Position des Nadelhalters ermittelt. Dies ist jedoch, wie gesagt, nicht unbedingt notwendig, da die Position (z. B. in einer Einsetzstellung) bereits bekannt sein kann.

Wurde der Abstand der Nadelspitze zu dem Bezugspunkt ermittelt, dann kann dieser sowohl zur Bestimmung der Nadellänge (bei einem Bezugspunkt am Nadelhalter) als auch zur Bestimmung der Nadelposition (bei einem festen Bezugspunkt im Raum z.B. an der Nadelführung) verwendet werden. Es wird dabei automatisch der Abstand von der Nadelspitze zu diesem Bezugspunkt ermittelt. Der Bezugspunkt ist bevorzugt ein Punkt am Nadelhalter, z.B. dessen Nadelhaltepunkt. Der Bezugspunkt kann aber auch die Nadelführung oder ein anderer Punkt außerhalb des Nadelhalters sein.

Die Berechnung der gesamten Nadellänge ist mit den in dem Verfahren bekannten Informationen einfach. Sie kann aus der Distanz zwischen Nadelspitze und Nadelhalter abgeleitet werden, z.B. aus einem Bild (hier wäre eine Kamera der Sensor). Zwar ist die Nadellänge im Bild nicht die der realen Nadel, jedoch kann bei einem vorgegebenen Aufnahmeabstand und ggf. einer Anzahl von Eichungsmessungen einfach ein Umrechnungsfaktor bestimmt werden. Es müssen also im Grunde nur die Pixel von Nadelspitze bis zum Nadelhalter gezählt werden. Aus dem Umrechnungsfaktor ergibt sich dann direkt die Länge der realen Nadel.

Was die Position der Nadel betrifft, muss der Abstand von der Nadelspitze bis zu einem (bekannten) Bezugspunkt berechnet werden, z.B. der Nadelführung oder dem Nadelhalter. Hier sollte beachtet werden, dass es nicht unbedingt notwendig ist, für die Ermittlung der Position der Nadelspitze der Biopsienadel unbedingt die Spitze zu vermessen. Es genügt dazu, dass die Position aus Messungen abgeleitet werden kann. Bevorzugt wird dazu initial (wenn die Nadelspitze sich noch außerhalb des Patienten befindet) die Position der Nadelspitze direkt ermittelt. Dies kann bevorzugt dadurch geschehen, dass ihre Position an der Nadelführung durch einen Sensor an der Nadelführung ermittelt wird. danach kann die Position des Nadelhalters gemessen werden und aus dessen Position (bei bekannter Nadellänge) die aktuelle Position der Nadelspitze abgeleitet (und dadurch ermittelt) werden.

Zur Messung der Nadellänge benötigt man im Grunde nur einen Durchlauf der Verfahrensschritte. Bei der Bestimmung der Position der Nadelspitze im Körper sollten die Verfahrensschritte mehrfach durchlaufen werden. Es ist besonders bevorzugt vor dem Einführen der Biopsienadel die Nadellänge zu bestimmen und damit zu überprüfen, ob die korrekte Nadel eingesetzt worden ist. Danach kann dann die Position der Nadelspitze im Körper ermittelt werden. Dazu könnten die Verfahrensschritte nach dem Positionieren der Biopsienadel im Nadelhalter wie folgt konkretisiert werden:
a) Ermitteln zumindest der Position der Nadelspitze der Biopsienadel mittels eines Sensors, insbesondere im Bereich der Nadelführung,
b) automatisches Berechnen einer Gesamt-Nadellänge von der Nadelspitze zu dem Nadelhalter als ersten Bezugspunkt,
c) Bestimmen der Position des Nadelhalters mittels eines Sensors und ermitteln der Position der Nadelspitze der Biopsienadel aus der Position des Nadelhalters und der berechneten Gesamt-Nadellänge,
d) automatisches Berechnen eines Abstandes von der Nadelspitze zu der Nadelführung als zweiten Bezugspunkt,
e) mehrfaches Wiederholen der Schritte c) und d).

Hierzu sollte beachtet werden, dass der mögliche Bewegungsvektor des Nadelhalters in der Biopsie-Einheit bekannt ist. Der Nadelhalter kann nur entlang dieses Bewegungsvektors (oder "Bewegungsrichtung") verschoben werden. Da die Biopsienadel starr ist, wird die Nadelspitze bei Verschiebung des Nadelhalters entsprechend verschoben. Ist also die Position eines Bezugspunktes am Nadelhalter bekannt und ist zudem der Abstand der Nadelspitze zu diesem Bezugspunkt bekannt, dann kann die Position der Nadelspitze entlang des Bewegungsvektors und damit auch im Raum exakt bestimmt werden.

Eine erfindungsgemäße Vorrichtung dient zur automatisierten Bestimmung der Position einer Nadelspitze einer Biopsienadel, welche in einem Nadelhalter angebracht ist und für eine Biopsie durch eine Biopsie-Einheit. auf ihrer longitudinalen Achse verschiebbar ist und mittels einer Nadelführung geführt wird. Die Vorrichtung umfasst die folgenden Komponenten:
- optional: einen Sensor zum Erkennen eines Positionierens einer Biopsienadel im Nadelhalter,
- eine Ermittlungseinheit, ausgelegt zum Ermitteln zumindest der Position der Nadelspitze der Biopsienadel mittels eines Sensors,
- eine Berechnungseinheit, ausgelegt zum automatisches Berechnen eines Abstandes von der Nadelspitze zu einem Bezugspunkt,
bevorzugt: eine Datenschnittstelle ausgelegt zum Ausgeben des Abstandes, insbesondere in Form einer Nadellänge oder einer Position der Nadelspitze.

Die Funktion der Komponenten der Vorrichtung wurde bereits vorangehend beschrieben. Die Vorrichtung ist bevorzugt zur Ausführung eines erfindungsgemäßen Verfahrens ausgelegt.

Ein erfindungsgemäßes Mammographiesystem umfasst eine Vorrichtung gemäß der Erfindung und/oder ist zum Durchführen eines Verfahrens gemäß der Erfindung ausgelegt.

Die Erfindung kann insbesondere in Form einer Rechnereinheit mit geeigneter Software realisiert sein. Die Rechnereinheit kann z.B. hierzu einen oder mehrere zusammenarbeitende Mikroprozessoren oder dergleichen aufweisen. Insbesondere kann sie in Form von geeigneten Softwareprogrammteilen in der Rechnereinheit realisiert sein. Eine weitgehend softwaremäßige Realisierung hat den Vorteil, dass auch schon bisher verwendete Rechnereinheiten auf einfache Weise durch ein Software- bzw. Firmware-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Rechnereinheit ladbar ist, mit Programmabschnitten, um alle Schritte des erfindungsgemäßen Verfahrens auszuführen, wenn das Programm in der Rechnereinheit ausgeführt wird. Ein solches Computerprogrammprodukt kann neben dem Computerprogramm gegebenenfalls zusätzliche Bestandteile wie z.B. eine Dokumentation und/oder zusätzliche Komponenten, auch Hardware-Komponenten, wie z.B. Hardware-Schlüssel (Dongles etc.) zur Nutzung der Software, umfassen.

Zum Transport zur Rechnereinheit und/oder zur Speicherung an oder in der Rechnereinheit kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Ein bevorzugter Sensor für das Ermitteln der Position der Nadelspitze der Biopsienadel im Rahmen des Verfahrens bzw. für eine bevorzugte Vorrichtung sind ein kapazitiver Sensor, ein induktiver Sensor, ein optischer Sensor oder ein akustischer Sensor. Dieser Sensor kann die Position der Nadelspitze direkt messen oder die Position des Nadelhalters. Bevorzugt misst dieser Sensor jedoch die Position der Nadelspitze (direkt). Diese Messung erfolgt bevorzugt mittels einem Sensor an oder in der Nadelführung.

Alternativ oder zusätzlich ist bevorzugt, dass ein optischer Sensor überwacht, ob die Biopsienadel eine vorgegebene Position erreicht hat. Hier sollte beachtet werden, dass sich die Nadelspitze stets in Verschiebungsrichtung des Nadelhalters bewegt. Ein optischer Sensor, z.B. eine Lichtschranke kann ermitteln, wann die Nadelspitze einen bestimmten Punkt auf der Verschiebungsrichtung erreicht hat. Da die Biopsienadel in der Regel metallisch und ggf. auch magnetisch ist, kann man die Anwesenheit der Nadel an einem bestimmten Punkt mit einem induktiven oder kapazitiven Sensor bestimmen. Auch eine Kamera ist als Sensor bevorzugt, wie weiter unten noch genauer ausgeführt wird.

Bevorzugt erfolgt zumindest in dem Fall, dass sich die Nadelspitze in einem Patienten befindet und/oder nach einer Bestimmung der Nadellänge oder zusätzlich zu einer (direkten) Messung der Nadelspitze eine Messung der Position des Nadelhalters. Ein bevorzugter Sensor dafür ist ein kapazitiver Sensor, ein induktiver Sensor, ein optischer Sensor, oder ein akustischer Sensor. Auch kann ein Potentiometer abgefragt werden bzw. dessen Zustand mit einem Sensor gemessen werden. Zudem ist es möglich, die Position des Nadelhalters auch aus einer Steuervorgabe zur Verschiebung des Nadelhalters zu bestimmen. Die Messung der Position des Nadelhalters in Ergänzung zur (direkten) Messung der Position der Nadelspitze hat den Vorteil, dass die Position der Nadelspitze ermittelt werden kann auch wenn die Nadelspitze sich in einem Patienten befindet.

Bevorzugt wird zunächst die Anwesenheit der Nadelspitze im Bereich der Nadelführung überprüft. Wurde die Nadelspitze dort erkannt, dann ist es nicht mehr unbedingt notwendig, dass die Position der Nadelspitze weiterhin ermittelt wird. Da die Nadellänge bekannt ist (entweder automatisch bestimmt oder da die eingesetzte Nadel bekannt und überprüft ist) und die Bewegungsrichtung der Nadel bekannt ist (entlang der Bewegungsrichtung), kann anhand der Ermittlung der Position des Nadelhalters die Position der Nadelspitze eindeutig bestimmt werden.

Es ist dabei bevorzugt, dass eine Verschiebung des Nadelhalters von einer Skala abgelesen oder mittels einer Skala ermittelt wird oder der Zustand eines Schiebeschlittens der Biopsie-Einheit ermittelt wird. Durch rechnerische Erfassung z.B. der Zählschritte bei Verwendung einer Skala oder des Analogwertes bei Anwendung analoger Mess-Systeme (z.B. Potentiometer) beginnend mit dem Einführen der Nadel in den Nadeldetektor oder durch Auslesen von festen Positionen kann mittels eines Kontrollsystem der Zustand erfasst werden oder kann auch durch Anzeige für den Benutzer an einem Display visualisiert werden. Im Weiteren kann durch ein programmierbares Kontrollsystem Position bzw. Nadellänge ausgewertet werden, ggf. die korrekte Position korrigiert werden oder zur Fehleranzeige gebracht werden. Eine geeignete Erfassung mit weniger hoher Genauigkeit kann z.B. mittels Detektion von wenigen Positionen z.B. 3er voreingestellter Längen erreicht werden.

Bevorzugt erfolgt vor einer Biopsie und nach dem Positionieren der Biopsienadel im Nadelhalter eine Messung der Länge der Biopsienadel. Eine Messung erfolgt dabei bevorzugt durch Aufnahme der Biopsienadel im Nadelhalter in einem Bild (insbesondere mittels einer Kamera) und Auswertung des Bildes. Alternativ kann dazu auch die Positionierung der Nadelspitze an der Nadelführung bestimmt werden und anschließend der Nadelhalter in Richtung der Nadelführung bis zu einem Anschlagspunkt verfahren werden (ohne Patient). Wie oben bereits gesagt hat die Messung der Länge der Biopsienadel den Vorteil, dass Fehler bei der Positionsbestimmung der Nadelspitze, die durch Abweichungen einer angegebenen Nadellänge von einer realen Nadellänge entstanden sind, vermieden werden.

Bevorzugt wird die Position der Nadelspitze der Biopsienadel mittels einem optischen Sensor, bevorzugt einer Kamera (2D oder 3D), ermittelt. Es ist dabei bevorzugt, dass ein Bild der Biopsienadel aus einer festgelegten Aufnahmeposition mit einem vorgegebenen Abstand zur Biopsienadel aufgenommen wird und eine Länge der Biopsienadel berechnet wird, indem in dem Bild die Biopsienadel und der Nadelhalter gesucht werden und die Länge der Biopsienadel im Bild (z.B. durch Zählen von Pixeln) mittels einer vorgegebenen Funktion (z.B. Triangulierung oder eine Funktion zur Kompensation einer bekannten Verzerrung im Bild) auf die wahre Länge der Biopsienadel umgerechnet wird. Bevorzugt wird zusätzlich auch die Dicke der Biopsienadel im Bild (z.B. auch durch Zählen von Pixeln ermittelt) mittels einer vorgegebenen Funktion auf die wahre Dicke der Biopsienadel umgerechnet. Dies hat den Vorteil, dass mit sehr wenig Aufwand und ohne eine genaue Position anfahren zu müssen, die Länge der Biopsienadel bestimmt werden kann. Hier sei darauf hingewiesen, dass die Nadeln lediglich in Form von (bekannten) Längenstufen vorliegen, die sich deutlich voneinander unterscheiden. Daher ist in der Regel eine millimetergenaue Bestimmung der Länge nicht unbedingt notwendig.

Es ist bevorzugt, dass bei einer Ausführungsform des Verfahrens das Vorhandensein einer Biopsienadel im Nadelhalter mittels eines Sensors ermittelt wird. Ein solcher Sensor kann z.B. ein Schalter sein, der aktiviert ist, wenn sich eine Biopsienadel im Nadelhalter befindet und deaktiviert ist, wenn nicht. In dem Falle, dass eine Biopsienadel eingesetzt ist, wird dann eine Verschiebung des Nadelhalters bei vorgegebenen Situationen blockiert, insbesondere wenn ein Abstand von der Nadelspitze zu einem festen Bezugspunkt einen vorgegebenen Grenzwert überschreitet. Dies verhindert, dass eine Biopsienadel über den Zielbereich in einem Patienten hinausdringt.

Bevorzugt werden nach dem Positionieren der Biopsienadel im Nadelhalter und bevorzugt auch nach einem optionalen Überprüfung, ob eine Biopsienadel im Nadelhalter eingesetzt ist, die folgenden Schritte durchlaufen:
- Aufnehmen eines Bildes von der Biopsienadel und dem Nadelhalter,
- Segmentieren des Bildes,
- Durchführen einer Konturdetektion zumindest der Biopsienadel,
- Zählen derjenigen Pixel, die als Biopsienadel segmentiert worden sind,
- Berechnen der Länge der realen Biopsienadel mittels der Länge der Biopsienadel im Bild und einer vorgegebenen Formel und bevorzugt auch der Dicke der realen Biopsienadel mittels der Dicke der Biopsienadel im Bild und einer vorgegebenen Formel,
- Ausgeben der berechneten Werte bevorzugt in Form eine Bildes mit entsprechenden Bema-ßungen.

Das Bild wird bevorzugt von einer Kamera aufgenommen, wie sie oben bereits beschrieben worden ist. Dabei ist es wichtig, dass die Biopsienadel komplett aufgenommen wird und von dem Nadelhalter zumindest der Teil, in dem die Biopsienadel befestigt ist.

Eine Segmentierung eines Bildes ist im Stand der Technik bekannt. Diese erfolgt bevorzugt mittels eines maschinenlernfähigen Modells, welches zur Erkennung von Biopsienadeln und Nadelhaltern in einem Bild trainiert worden ist. Nach dieser Segmentierung ist bekannt, welche Pixel des Bildes die Biopsienadel darstellen und welche den Nadelhalter.

Das Durchführen einer Konturdetektion von segmentierten Elementen (also auch der Biopsienadel) ist im Stand der Technik bekannt. Bevorzugt erfolgt diese mittels einer Minimalrechteck-Methode, die ebenfalls im Stand der Technik bekannt ist. Es wird dabei das kleinste Rechteck um die betreffende Kontur herum gesucht. Damit sind diejenigen Pixel, welche die Biopsienadel darstellen separiert.

Diese separierten Pixel können dann gezählt werden. Am einfachsten ist es, wenn die Aufnahme so justiert ist, dass die Nadel genau in X- oder Y-Richtung des Bildes verläuft. Aber auch schräge Darstellungen sind durchaus möglich. Bevorzugt erfolgt das Zählen zumindest in vertikaler Richtung. Das Zählen dient zur Ermittlung der Länge der Biopsienadel im Bild. Vorzugsweise werden Pixel auch zusätzlich in horizontaler Richtung gezählt. Dies dient zur Ermittlung der Dicke der Biopsienadel im Bild.

Ist die Zahl der Pixel bekannt, erfolgt die Berechnung der Länge der realen Biopsienadel. Da die Biopsienadel im Bild eine andere Länge hat als in der Realität, diese aber lediglich durch eine bekannte (bzw. ermittelbare) Funktion skaliert ist, kann mittels der Länge der Biopsienadel im Bild und einer vorgegebenen Formel die reale Länge berechnet werden. In dem Falle, dass ein linearer Zusammenhang besteht, wäre die Reale Länge L = aB mit dem Faktor a und der Länge B im Bild. Bevorzugt wird auch die Dicke der realen Biopsienadel mittels der Dicke der Biopsienadel im Bild und einer vorgegebenen Formel berechnet. Dies kann entsprechend wie die Länge erfolgen. Hierzu sei aber angemerkt, dass die Biopsienadel häufig schräg angeordnet ist. Dies hat sowohl Auswirkungen auf die Berechnung der Länge als auch auf die Dicke (hier wäre die Nadel leicht kegelförmig). In der Praxis hat dies jedoch nur geringe Auswirkungen, da die Schrägstellung für die Berechnung der Länge durch geeignete Wahl der Funktion kompensiert werden kann. Was die Dicke betrifft, kann ein bestimmter Punkt der Nadel gewählt werden (z.B. die Spitze).

Die berechneten Werte können dann z.B. dargestellt bzw. in einem Logfile abgespeichert werden. Bevorzugt ist eine Ausgabe des Bildes der Nadel mit entsprechenden Bemaßungen.

Gemäß einer bevorzugten Ausführungsform des Verfahrens wird zusätzlich der Nadelhalter nach Merkmalen untersucht, die dessen Hersteller offenbaren. Bevorzugt wird dabei im Bild an der Position des Nadelhalters nach Zeichen gesucht, insbesondere in Form von Logos und/oder Schriftzeichen. Gefundene Zeichen werden dann mit einer Liste aus Zeichen verglichen. Dies dient der besseren Überprüfung, ob eine korrekte Nadel verwendet wird.

Eine bevorzugte Vorrichtung umfasst einen optischen Sensor ausgelegt und angeordnet zur Aufnahme von Bildern des Nadelhalters und einer eingelegten Biopsienadel. Zusätzlich umfasst die Vorrichtung eine Längenermittlungseinheit, die zum Ermitteln der Länge der Biopsienadel ausgelegt ist. Sie ist besonders bevorzugt dazu ausgelegt, in dem Bild die Biopsienadel und den Nadelhalter zu suchen und die Länge der Biopsienadel im Bild mittels einer vorgegebenen Funktion auf die wahre Länge der Biopsienadel umzurechnen. Bevorzugt ist sie auch dazu ausgelegt, die Dicke der Biopsienadel im Bild mittels einer vorgegebenen Funktion auf die wahre Dicke der Biopsienadel umzurechnen.

Gemäß einer bevorzugten Ausführungsform des Verfahrens wird vor Einsetzen der Biopsienadel in den Nadelhalter ein Identifikationscode, z.B. ein QR-Code, ein Label, eine Zeichenkette, eine NFC-Information oder ein Barcode, an der Biopsienadel oder einer Verpackung der Biopsienadel gescannt. Die gescannte Information wird dann mit einer vorgegebenen Information über einen gewünschten Nadeltyp verglichen. In dem Fall, dass der Identifikationscode anzeigt, dass es sich nicht um den gewünschten Nadeltyp handelt, wird eine Warninformation ausgegeben. Dies hat den Vorteil, dass ein fälschliches Aufreißen der Verpackung einer sterilen Nadel verhindert werden kann. Alternativ oder zusätzlich wird ein Einsetzen der Biopsienadel in den Nadelhalter blockiert. Dies hat den Vorteil, dass neben der menschlichen Aufmerksamkeit noch eine weitere Sicherung gegen die Verwendung einer falschen Nadel existiert.

Eine bevorzugte Vorrichtung umfasst einen Sensor ausgelegt zur Detektion der Nadelspitze an der Nadelführung und einen Sensor ausgelegt zur Bestimmung der Position des Nadelhalters. Es ist dabei bevorzugt, dass die Berechnungseinheit dazu ausgelegt ist, basierend auf der gemessenen Position der Nadelspitze an der Nadelführung und der Position des Nadelhalters automatisch einen Abstand von der Nadelspitze zu einem festen Bezugspunkt im Raum zu berechnen, bevorzugt zu einem Punkt am Nadelhalter.

Es kann gewünscht sein, nur die Nadellänge zu bestimmen, um zu überprüfen, ob die korrekte Biopsienadel vorliegt. Ein hierzu bevorzugtes Verfahren dient zur automatisierten Bestimmung der Länge einer Biopsienadel. Das Verfahren umfasst die folgenden Schritte:
- Bereitstellen einer Biopsienadel (in ihrer Verpackung oder im Nadelhalter),
- Aufnahme eines Bildes der Biopsienadel (in dem Nadelhalter oder in ihrer Verpackung) mit einem optischen Sensor, insbesondere einer Kamera oder einem Code-Lesegerät, oder Aufnehmen einer NFC-Information der Nadel mittels eines Sensors,
- Ermitteln der Nadellänge aus der Information des Sensors, insbesondere der Bildinformationen des Bildes,
- Ausgeben der Nadellänge (als Information an einen Bediener oder in Form von Steuerbefehlen).

Es sind dabei zwei Ausführungsformen des Verfahrens besonders bevorzugt. Zum einen kann mit diesem Verfahren ein Code (z.B. Barcode oder QR-Code) an der Biopsienadel oder einer Verpackung gelesen werden und überprüft werden, ob eine korrekte (verpackte) Biopsienadel ausgewählt worden ist. Diese Ausführungsform kann verhindern, dass fälschlicher Weise eine falsche Biopsienadel eingesetzt wird, die in diesem Falle weggeworfen werden müsste, da sie nicht mehr steril wäre. Damit verhindert diese Ausführungsform unnötige Kosten.

Die andere bevorzugte Ausführungsform erlaubt eine automatische Bestimmung der Nadellänge bei einer eingesetzten Nadel aus den Bildern. Diese wurde bereits oben beschrieben.

Zunächst wird die Biopsienadel bereitgestellt. Sie kann in ihrer Verpackung vorliegen (für das eine bevorzugte Verfahren) oder bereits im Nadelhalter (für das andere bevorzugte Verfahren).

Es wird dann ein Bild der Biopsienadel aufgenommen. Für das eine bevorzugte Verfahren kann dies das Bild eines Lesegerätes für Barcodes oder QR-Codes sein, für das andere bevorzugte Verfahren ein Bild einer Kamera wie oben beschrieben.

Aus diesem Bild kann dann die Nadellänge ermittelt werden. Bei dem einen bevorzugten Verfahren kann die Nadellänge direkt aus der Information des Codes herausgelesen werden, bei dem anderen bevorzugten Verfahren durch Auswertung der Bildinformation (z.B. Zählen von Pixeln) wie oben bereits beschrieben.

Die ermittelte Nadellänge kann dann als Information an einen Bediener oder in Form von Steuerbefehlen ausgegeben werden.

Eine diesbezüglich bevorzugte Vorrichtung zur automatisierten Bestimmung der Länge einer Biopsienadel, insbesondere mit dem vorangehend beschriebenen Verfahren, umfasst die folgenden Komponenten:
- Bereitstellen einer Biopsienadel (in ihrer Verpackung oder im Nadelhalter),
- einen optischen Sensor ausgelegt zur Aufnahme eines Bildes der Biopsienadel, insbesondere eine Kamera oder ein Code-Lesegerät,
- eine Längenermittlungseinheit ausgelegt zum Ermitteln der Nadellänge aus Bildinformationen des Bildes,
- eine Datenschnittstelle ausgelegt zum Ausgeben der Nadellänge (als Information an einen Bediener oder in Form von Steuerbefehlen).

Die Funktion der Komponenten der Vorrichtung wurde bereits vorangehend beschrieben. Die Vorrichtung ist bevorzugt zur Ausführung eines erfindungsgemäßen Verfahrens ausgelegt.

Ein bevorzugtes Mammographiesystem umfasst diese Vorrichtung und/oder ist zum Durchführen des bevorzugten Verfahrens ausgelegt.

Ein bevorzugter Sensor für das bevorzugte Verfahren bzw. die bevorzugte Vorrichtung zur Längenmessung wurde oben bereits genannt. Gleiches gilt für die Überwachung, ob die Biopsienadel eine vorgegebene Position erreicht hat.

Eine bevorzugte Messung der Nadellänge erfolgt bevorzugt durch Aufnahme der Biopsienadel im Nadelhalter in einem Bild (insbesondere mittels einer Kamera) und Auswertung des Bildes. Alternativ kann dazu auch die Positionierung der Nadelspitze an der Nadelführung bestimmt werden und anschließend der Nadelhalter in Richtung der Nadelführung bis zu einem Anschlagspunkt verfahren werden (ohne Patient). Wie oben bereits gesagt hat die Messung der Länge der Biopsienadel den Vorteil, dass Fehler bei der Positionsbestimmung der Nadelspitze, die durch Abweichungen einer angegebenen Nadellänge von einer realen Nadellänge entstanden sind, vermieden werden.

Bevorzugt wird, wie gesagt, die Nadellänge mittels einem optischen Sensor ermittelt, bevorzugt einer Kamera (2D oder 3D). Es ist dabei bevorzugt, dass ein Bild der Biopsienadel aus einer festgelegten Aufnahmeposition mit einem vorgegebenen Abstand zur Biopsienadel aufgenommen wird und eine Länge der Biopsienadel berechnet wird, indem in dem Bild die Biopsienadel und der Nadelhalter gesucht werden und die Länge der Biopsienadel im Bild (z.B. durch Zählen von Pixeln) mittels einer vorgegebenen Funktion (z.B. Triangulierung oder eine Funktion zur Kompensation einer bekannten Verzerrung im Bild) auf die wahre Länge der Biopsienadel umgerechnet wird. Bevorzugt wird zusätzlich auch die Dicke der Biopsienadel im Bild (z.B. auch durch Zählen von Pixeln ermittelt) mittels einer vorgegebenen Funktion auf die wahre Dicke der Biopsienadel umgerechnet. Dies hat den Vorteil, dass mit sehr wenig Aufwand und ohne eine genaue Position anfahren zu müssen, die Länge der Biopsienadel bestimmt werden kann. Hier sei darauf hingewiesen, dass die Nadeln lediglich in Form von (bekannten) Längenstufen vorliegen, die sich deutlich voneinander unterscheiden. Daher ist in der Regel eine millimetergenaue Bestimmung der Länge nicht unbedingt notwendig.

Bevorzugt werden nach dem Positionieren der Biopsienadel im Nadelhalter und bevorzugt auch nach einer optionalen Überprüfung, ob eine Biopsienadel im Nadelhalter eingesetzt ist, die folgenden Schritte durchlaufen:
- Aufnahme eines Bildes von der Biopsienadel und dem Nadelhalter,
- Segmentierung des Bildes,
- Durchführen einer Konturdetektion zumindest der Biopsienadel,
- Zählen derjenigen Pixel, die als Biopsienadel segmentiert worden sind,
- Berechnung der Länge der realen Biopsienadel mittels der Länge der Biopsienadel im Bild und einer vorgegebenen Formel und bevorzugt auch der Dicke der realen Biopsienadel mittels der Dicke der Biopsienadel im Bild und einer vorgegebenen Formel,
- Ausgabe der berechneten Werte bevorzugt in Form eines Bildes mit entsprechenden Bema-ßungen.

Das Bild wird bevorzugt von einer Kamera aufgenommen, wie sie oben bereits beschrieben worden ist. Dabei ist es wichtig, dass die Biopsienadel komplett aufgenommen wird und von dem Nadelhalter zumindest der Teil, in dem die Biopsienadel befestigt ist.

Eine Segmentierung eines Bildes ist im Stand der Technik bekannt. Diese erfolgt bevorzugt mittels eines maschinenlernfähigen Modells, welches zur Erkennung von Biopsienadeln und Nadelhaltern in einem Bild trainiert worden ist. Nach dieser Segmentierung ist bekannt, welche Pixel des Bildes die Biopsienadel darstellen und welche den Nadelhalter.

Das Durchführen einer Konturdetektion von segmentierten Elementen (also auch der Biopsienadel) ist im Stand der Technik bekannt. Bevorzugt erfolgt diese mittels einer Minimalrechteck-Methode, die ebenfalls im Stand der Technik bekannt ist. Es wird dabei das kleinste Rechteck um die betreffende Kontur herum gesucht. Damit sind diejenigen Pixel, welche die Biopsienadel darstellen separiert.

Diese separierten Pixel können dann gezählt werden. Am einfachsten ist es, wenn die Aufnahme so justiert ist, dass die Nadel genau in X- oder Y-Richtung des Bildes verläuft. Aber auch schräge Darstellungen sind durchaus möglich. Bevorzugt erfolgt das Zählen zumindest in vertikaler Richtung. Das Zählen dient zur Ermittlung der Länge der Biopsienadel im Bild. Vorzugsweise werden Pixel auch zusätzlich in horizontaler Richtung gezählt. Dies dient zur Ermittlung der Dicke der Biopsienadel im Bild.

Ist die Zahl der Pixel bekannt, erfolgt die Berechnung der Länge der realen Biopsienadel. Da die Biopsienadel im Bild eine andere Länge hat als in der Realität, diese aber lediglich durch eine bekannte (bzw. ermittelbare) Funktion skaliert ist, kann mittels der Länge der Biopsienadel im Bild und einer vorgegebenen Formel die reale Länge berechnet werden. In dem Falle, dass ein linearer Zusammenhang besteht, wäre die Reale Länge L = aB mit dem Faktor a und der Länge B im Bild. Bevorzugt wird auch die Dicke der realen Biopsienadel mittels der Dicke der Biopsienadel im Bild und einer vorgegebenen Formel berechnet. Dies kann entsprechend wie die Länge erfolgen. Hierzu sei aber angemerkt, dass die Biopsienadel häufig schräg angeordnet ist. Dies hat sowohl Auswirkungen auf die Berechnung der Länge als auch auf die Dicke (hier wäre die Nadel leicht kegelförmig). In der Praxis hat dies jedoch nur geringe Auswirkungen, da die Schrägstellung für die Berechnung der Länge durch geeignete Wahl der Funktion kompensiert werden kann. Was die Dicke betrifft, kann ein bestimmter Punkt der Nadel gewählt werden (z.B. die Spitze).

Die berechneten Werte können dann z.B. dargestellt bzw. in einem Logfile abgespeichert werden. Bevorzugt ist eine Ausgabe des Bildes der Nadel mit entsprechenden Bemaßungen.

Gemäß einer bevorzugten Ausführungsform des Verfahrens wird zusätzlich der Nadelhalter nach Merkmalen untersucht, die dessen Hersteller offenbaren. Bevorzugt wird dabei im Bild an der Position des Nadelhalters nach Zeichen gesucht, insbesondere in Form von Logos und/oder Schriftzeichen. Gefundene Zeichen werden dann mit einer Liste aus Zeichen verglichen. Dies dient der besseren Überprüfung, ob eine korrekte Nadel verwendet wird.

Eine bevorzugte Vorrichtung umfasst, wie gesagt, einen optischen Sensor ausgelegt und angeordnet zur Aufnahme von Bildern des Nadelhalters und einer eingelegten Biopsienadel. Zusätzlich umfasst die Vorrichtung eine Längenermittlungseinheit, die zum Ermitteln der Länge der Biopsienadel ausgelegt ist. Sie ist besonders bevorzugt dazu ausgelegt, in dem Bild die Biopsienadel und den Nadelhalter zu suchen und die Länge der Biopsienadel im Bild mittels einer vorgegebenen Funktion auf die wahre Länge der Biopsienadel umzurechnen. Bevorzugt ist sie auch dazu ausgelegt, die Dicke der Biopsienadel im Bild mittels einer vorgegebenen Funktion auf die wahre Dicke der Biopsienadel umzurechnen.

Gemäß einer bevorzugten Ausführungsform des Verfahrens wird vor Einsetzen der Biopsienadel in den Nadelhalter ein Identifikationscode, z.B. ein QR-Code, ein Label, eine Zeichenkette, eine NFC-Information oder ein Barcode, an der Biopsienadel oder einer Verpackung der Biopsienadel gescannt. Die gescannte Information wird dann mit einer vorgegebenen Information über einen gewünschten Nadeltyp verglichen. In dem Fall, dass der Identifikationscode anzeigt, dass es sich nicht um den gewünschten Nadeltyp handelt, wird eine Warninformation ausgegeben. Dies hat den Vorteil, dass ein fälschliches Aufreißen der Verpackung einer sterilen Nadel verhindert werden kann. Alternativ oder zusätzlich wird ein Einsetzen der Biopsienadel in den Nadelhalter blockiert. Dies hat den Vorteil, dass neben der menschlichen Aufmerksamkeit noch eine weitere Sicherung gegen die Verwendung einer falschen Nadel existiert.

Bevorzugt ist der Einsatz von Ki-basierten Verfahren (KI: "Künstliche Intelligenz") für das erfindungsgemäße Verfahren. Eine künstliche Intelligenz basiert auf dem Prinzip des maschinenbasierten Lernens, und wird in der Regel mit einem lernfähigen Algorithmus durchgeführt der entsprechend trainiert worden ist. Für maschinenbasiertes Lernen wird häufig der englische Ausdruck "Machine Learning" verwendet, wobei hier auch das Prinzip des "Deep Learning" mit umfasst wird. Besonders das Finden der Biopsienadel in Bildern ist eine Aufgabe für ein solches System.

Bevorzugt liegen Komponenten der Erfindung als ein "Cloud-Dienst" vor. Ein solcher Cloud-Dienst dient der Bearbeitung von Daten, insbesondere mittels einer künstlichen Intelligenz, kann aber auch ein Dienst basierend auf herkömmlichen Algorithmen sein oder ein Dienst, bei dem im Hintergrund eine Auswertung durch Menschen stattfindet. Generell ist ein Cloud-Dienst (im Folgenden auch kurz als "Cloud" bezeichnet) eine IT-Infrastruktur, bei der über ein Netzwerk z.B. Speicherplatz oder Rechenleistung und/oder eine Anwendungssoftware zur Verfügung gestellt wird. Die Kommunikation zwischen dem Anwender und der Cloud erfolgt dabei mittels Datenschnittstellen und/oder Datenübertragungsprotokollen. Im hier vorliegenden Fall ist besonders bevorzugt, dass der Cloud-Dienst sowohl Rechenleistung als auch Anwendungssoftware zur Verfügung stellt.

Im Rahmen eines bevorzugten Verfahrens erfolgt eine Bereitstellung von Daten, die im Rahmen der Erfindung erhalten werden, über das Netzwerk an den Cloud-Dienst. Dieser umfasst ein Rechensystem, das in der Regel nicht den lokalen Rechner des Benutzers umfasst. Das Verfahren kann dabei mittels einer Befehlskonstellation in einem Netzwerk realisiert werden. Die in der Cloud berechneten Daten werden später wieder über das Netzwerk zu dem lokalen Rechner des Anwenders gesendet.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine grob schematische Darstellung eines bevorzugten Mammographiesystems mit einer bevorzugten Vorrichtung,
Figur 2 eine Biopsie-Einheit
Figur 3 eine bevorzugte Sensoranordnung an einem mit einer Biopsienadel bestückten Nadelhalter,
Figur 4 eine weitere bevorzugte Sensoranordnung an einem mit einer Biopsienadel bestückten Nadelhalter,
Figur 5 die Messung der Nadellänge mit einer Kamera,
Figur 6 den Ablauf des Verfahrens als Blockschaltbild,
Figur 7 ein Scannen eines Barcodes zur Ermittlung des Nadeltyps.

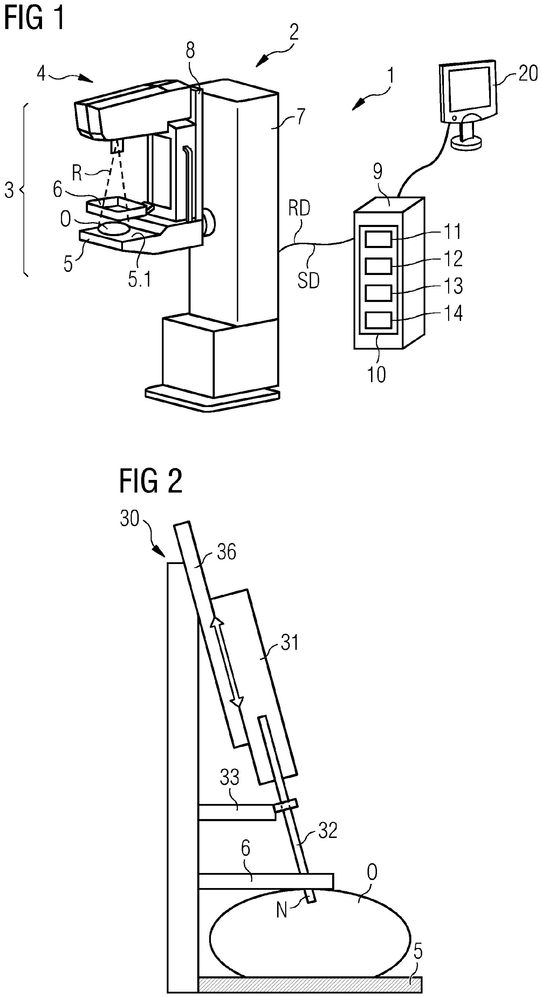

In Figur 1 ist beispielhaft und grob schematisch ein Mammographiesystem 1 in Form eines Tomosynthesesystems 1 gezeigt. Relative Richtungsangaben wie "oben", "unten" etc. beziehen sich auf ein bestimmungsgemäß für den Betrieb aufgestelltes Tomosynthesesystem 1. Das Tomosynthesesystem 1 umfasst ein Tomosynthesegerät 2 und eine Steuereinrichtung 9.

Das Tomosynthesegerät 2 weist eine Standsäule 7 und eine Quelle-Detektor-Anordnung 3 auf, die wiederum eine Röntgenstrahler 4 und einen Detektor 5 mit einer Detektorfläche 5.1 umfassen. Die Standsäule 7 steht im Betrieb auf dem Untergrund. Mit ihr ist die Quelle-Detektor-Anordnung 3 verschiebbar verbunden, sodass die Höhe der Detektorfläche 5.1, also der Abstand zum Untergrund, auf eine Brusthöhe einer Patientin eingestellt werden kann.

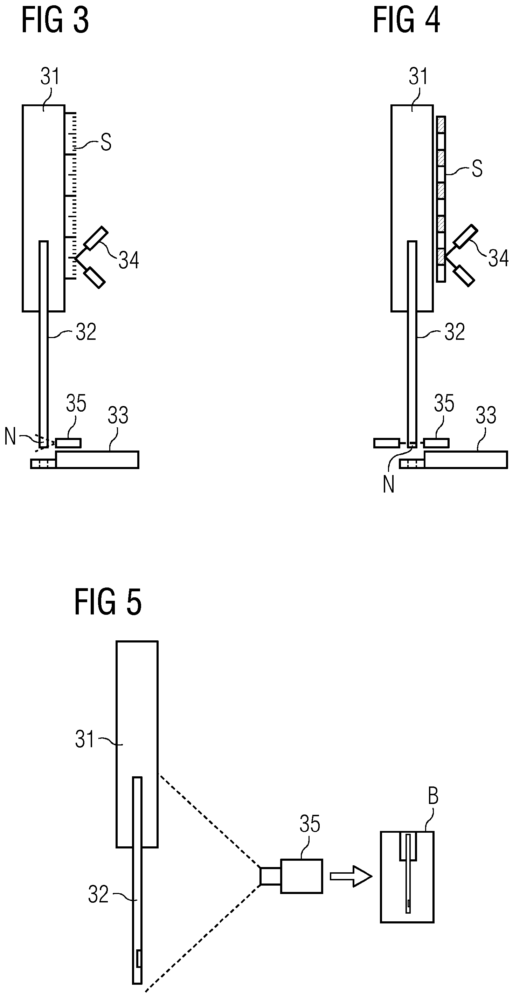

Eine Brust O der Patientin (hier schematisch dargestellt) liegt als Untersuchungsobjekt O für eine Untersuchung oberseitig auf der Detektorfläche 5.1 auf. Über der Brust O und der Detektorfläche 5.1 ist eine Kompressionsplatte 6 angeordnet, die verschiebbar mit der Quelle-Detektor-Anordnung 3 verbunden ist. Für die Untersuchung wird die Brust O komprimiert und zugleich fixiert, indem die Kompressionsplatte 6 auf sie herabgesenkt wird, sodass auf die Brust O zwischen Kompressionsplatte 6 und Detektorfläche 5.1 ein Druck ausgeübt wird. Die der Brust O zugewandte Berührungsoberfläche der Kompressionsplatte 6 weist eine konkave Wölbung auf, so dass die Brust dort konvex gewölbt ist, wie z.B. in den Figuren 4, 5 und 6 dargestellt ist.

Der Röntgenstrahler 4 ist dem Detektor 5 gegenüberliegend so angeordnet und ausgebildet, dass der Detektor 5 von ihm emittierte Röntgenstrahlung R erfasst, nachdem zumindest ein Teil der Röntgenstrahlung R die Brust O der Patientin durchdrungen hat. Dabei ist der Röntgenstrahler 4 relativ zum Detektor 5 mittels eines Dreharms 8 in einem Bereich von ± 50° um eine Grundstellung schwenkbar, in der sie senkrecht über der Detektorfläche 5.1 steht. Der aufzunehmende Ausschnitt kann mittels eines Kollimators C vorgegeben bzw. eingeschränkt werden.

Die Steuereinrichtung 9 erhält die Rohdaten RD der Messung und sendet Steuerdaten SD an das Tomosynthesegerät 2 mittels einer Datenschnittstelle. Sie ist mit einem Terminal 20 verbunden, über den ein Benutzer dem Tomosynthesesystem 1 Befehle mitteilen oder Messergebnisse abrufen kann. Die Steuereinrichtung 9 kann in demselben Raum wie das Tomosynthesegerät 2 angeordnet sein, es kann sich aber auch in einem angrenzenden Kontrollraum oder in einer noch weiteren räumlichen Entfernung befinden.

Die erfindungsgemäße Vorrichtung 10 dient zur automatisierten Bestimmung der Position einer Nadelspitze N einer Biopsienadel 32, welche in einem Nadelhalter 31 angebracht ist und für eine Biopsie durch eine Biopsie-Einheit 30 auf ihrer longitudinalen Achse verschiebbar ist und mittels einer Nadelführung 33 geführt wird (s. Figur 2). Sie umfasst eine Ermittlungseinheit 11, eine Berechnungseinheit 12 und eine Längenermittlungseinheit 13. Bevorzugt ist auch ein Sensor zum Erkennen eines Positionierens einer Biopsienadel 32 im Nadelhalter 31 (nicht dargestellt). Die Vorrichtung ist hier in der Steuereinrichtung angeordnet. Sie kann aber auch an anderen Stellen sinnvoll sein, z.B. am Dreaharm 8 oder an einem anderen Ort in der Nähe des Röntgenstrahls R oder sogar direkt an der Biopsie-Einheit 30.

Die Ermittlungseinheit 11 dient zum Ermitteln zumindest der Position der Nadelspitze N der Biopsienadel 32 mittels eines Sensors 35 (s. Nachfolgende Figuren).

Die Berechnungseinheit 12 dient zum automatisches Berechnen eines Abstandes von der Nadelspitze N zu einem Bezugspunkt.

Die Längenermittlungseinheit 13 dient zum Ermitteln der Länge der Biopsienadel 32, bevorzugt indem in dem Bild B die Biopsienadel 32 und der Nadelhalter 31 gesucht werden und die Länge der Biopsienadel 32 im Bild B mittels einer vorgegebenen Funktion auf die wahre Länge der Biopsienadel 32 umgerechnet wird. Sie kann auch zusätzlich dazu dienen, die Dicke der Biopsienadel 32 im Bild B mittels einer vorgegebenen Funktion auf die wahre Dicke der Biopsienadel 32 umzurechnen.

Über eine Datenschnittstelle 14 wird dann das Ergebnisbild E ausgegeben.

Figur 2 zeigt eine Biopsie-Einheit 30 mit einem Nadelhalter 31, der auf einem Schiebeschlitten 36 angeordnet ist. In den Nadelhalter 31 ist eine Biopsienadel 32 eingesetzt, die von einer Nadelführung 33 geführt wird. Die Biopsienadel 32 wurde hier zur Entnahme einer Biopsie in ein Untersuchungsobjekt O, hier ein weibliche Brust O, eingeführt. Die Brust O liegt dabei auf einem Detektor 5 eines Mammographiesystems 1 gemäß Figur 1 und die Biopsienadel 32 wurde durch ein Loch in der Kompressionsplatte 6 hindurchgeschoben. Ein Doppelpfeil deutet die mögliche Bewegungsrichtung von Biopsienadel 32 und Nadelhalter 31 an.

Figuren 3 und 4 zeigen eine bevorzugte Sensoranordnung an einem mit einer Biopsienadel 32 bestückten Nadelhalter 31 einer Biopsie-Einheit 30 nach Figur 2. Beide Ausführungsformen zeigen jeweils einen Sensor 34, der die Bewegung des Nadelhalters überwacht und einen Sensor 35 an der Nadelführung 33, der die Position der Nadelspitze N detektieren soll.

In Figur 3 ist an dem Nadelhalter eine Skala S angebracht, deren Position von einem optischen Sensor 34 überwacht wird. Damit kann sehr genau die Position des Nadelhalters 31 ermittelt werden, Da die Konfiguration von Nadelhalter 31 und Biopsienadel 32 starr ist, kann aus der Position des Nadelhalters 31 direkt auf die Position der Biopsienadel 32 geschlossen werden. Es wird jedoch zur Sicherheit noch die Nadelspitze N am Nadelhalter 35 ermittelt. Dies dient zur Sicherheit, dass eine bestimmte Position auf der Skala auf wirklich mit einer (ermittelten) Position der Nadelspitze N übereinstimmt. Wenn z.B. eine falsche Nadellänge ausgewählt worden ist, würde dies dann festgestellt werden. Der Nadelhalter 31 kann dazu in eine vorgegebene, hintere Position verschoben werden (wo die Nadelspitze N noch nicht am Nadelhalter 33 erkennbar ist und dann langsam nach vorne verschoben werden, bis der Sensor 35 am Nadelhalter 33 die Nadelspitze N detektiert. Nun kann direkt die Position der Skala S mit der Position der Nadelspitze N korreliert werden und sogar die Nadellänge ermittelt werden. Der hier dargestellte Sensor kann z.B. ein induktiver, kapazitiver oder akustischer Sensor sein.

In Figur 4 ist an dem Nadelhalter eine Skala S aus Schattierungen angebracht, deren Position von einem optischen Sensor 34 überwacht wird. Auch damit kann sehr genau die Position des Nadelhalters 31 ermittelt werden, insbesondere wenn der Sensor 34 eine räumliche Auflösung hat. Auch hier wird zur Sicherheit noch die Nadelspitze N am Nadelhalter 35 ermittelt, in diesem Beispiel mit einem optischen Sensor 35 in Form einer Lichtschranke.

Figur 5 zeigt das Ermitteln der Position der Nadelspitze der Biopsienadel 32 mittels einem optischen Sensor 35 in Form einer Kamera. In diesem Beispiel wird ein Bild B der Biopsienadel 32 aus einer festgelegten Aufnahmeposition mit einem vorgegebenen Abstand zur Biopsienadel 32 aufgenommen. Aus diesem Bild wird dann eine Länge der Biopsienadel 32 berechnet, indem in dem Bild B die Biopsienadel 32 und der Nadelhalter 31 gesucht werden und die Länge der Biopsienadel 32 im Bild B mittels einer vorgegebenen Funktion auf die wahre Länge der Biopsienadel 32 umgerechnet wird.

Dies erfolgt bevorzugt mittels der Schritte:
- Aufnehmen eines Bildes B von der Biopsienadel 32 und dem Nadelhalter 31,
- Segmentieren des Bildes B, bevorzugt mittels eines maschinenlernfähigen Modells trainiert zur Erkennung von Biopsienadeln 32 und Nadelhaltern 31 in einem Bild B,
- Durchführen einer Konturdetektion zumindest der Biopsienadel 32, bevorzugt mittels einer Minimalrechteck-Methode
- Zählen derjenigen Pixel, die als Biopsienadel 32 segmentiert worden sind, bevorzugt zumindest in vertikaler Richtung zur Ermittlung der Länge der Biopsienadel 32 im Bild B, vorzugsweise zusätzlich in horizontaler Richtung zur Ermittlung der Dicke der Biopsienadel 32 im Bild B,
- Berechnen der Länge der realen Biopsienadel 32 mittels der Länge der Biopsienadel 32 im Bild B und einer vorgegebenen Formel und bevorzugt auch der Dicke der realen Biopsienadel 32 mittels der Dicke der Biopsienadel 32 im Bild B und einer vorgegebenen Formel,
- Ausgeben der berechneten Werte bevorzugt in Form eine Bildes B mit entsprechenden Bema-ßungen.

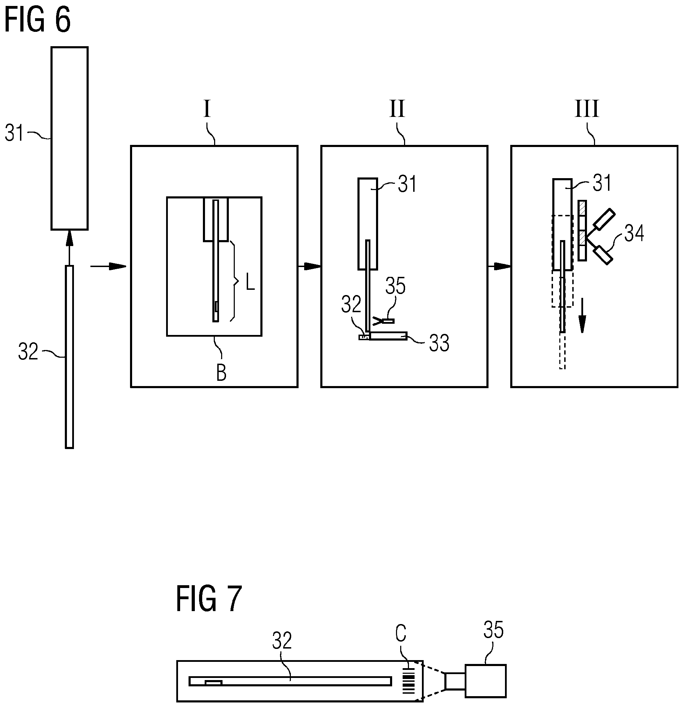

Figur 6 zeigt den Ablauf des Verfahrens zur automatisierten Bestimmung der Position einer Nadelspitze einer Biopsienadel mit einer Biopsie-Einheit nach Figur 2 als Blockschaltbild.

Zunächst wird dabei eine Biopsienadel 32 im Nadelhalter 31 positioniert (links).

In Schritt I wird dann zunächst aus einem Bild wie zu Figur 5 beschrieben die Länge L der Biopsienadel 32 ermittelt.

In Schritt II erfolgt ein Ermitteln zumindest der Position der Nadelspitze N der Biopsienadel 32 an der Nadelführung 33 mittels eines Sensors 35,

In Schritt III erfolgt ein automatisches Berechnen eines Abstandes von der Nadelspitze N zu der Nadelführung 33 als Bezugspunkt. Dabei wird mit einem Sensor 34 die Position des Nadelhalters 31 bestimmt und mit der ermittelten Länge L der Biopsienadel 32 auf deren genaue Position geschlossen.

Figur 7 zeigt ein Scannen eines Barcodes zur Ermittlung des Nadeltyps. Dabei wird vor dem Einsetzen der Biopsienadel 32 in den Nadelhalter 31 ein Barcode an der Biopsienadel 32 oder einer Verpackung der Biopsienadel 32 gescannt. Die gescannte Information wird dann mit einer vorgegebenen Information über einen gewünschten Nadeltyp verglichen. In dem Fall, dass der Barcode C anzeigt, dass es sich nicht um den gewünschten Nadeltyp handelt, wird eine Warninformation ausgegeben.

Es wird abschließend noch einmal darauf hingewiesen, dass es sich bei der vorhergehend detailliert beschriebenen Erfindung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schlie-ßen Begriffe wie "Einheit" nicht aus, dass die betreffenden Komponenten aus mehreren zusammenwirkenden Teil-Komponenten bestehen, die ggf. auch räumlich verteilt sein können. Der Begriff "eine Anzahl" ist als "mindestens ein(e)" zu lesen. Unabhängig vom grammatikalischen Geschlecht eines bestimmten Begriffes sind Personen mit männlicher, weiblicher oder anderer Geschlechteridentität mit umfasst.

## Patentansprüche

1. Verfahren zur automatisierten Bestimmung der Position einer Nadelspitze (N) einer Biopsienadel (32), welche in einem Nadelhalter (31) angebracht ist und für eine Biopsie durch eine Biopsie-Einheit (30) auf ihrer longitudinalen Achse verschiebbar ist und mittels einer Nadelführung (33) geführt wird, das Verfahren umfassend die Schritte:
- Positionieren der Biopsienadel (32) im Nadelhalter (31),
- Ermitteln zumindest der Position der Nadelspitze (N) der Biopsienadel (32) mittels eines Sensors (35),
- automatisches Berechnen eines Abstandes von der Nadelspitze (N) zu einem Bezugspunkt.

2. Verfahren nach Anspruch 1, wobei das Ermitteln der Position der Nadelspitze (N) der Biopsienadel (32) mittels einem kapazitiven Sensor, einem induktiven Sensor, einem optischen Sensor, oder einem akustischen Sensor erfolgt, bevorzugt mittels einem Sensor (35) an oder in der Nadelführung (33) und/oder einem optischen Sensor (35) der überwacht, ob die Biopsienadel (32) eine vorgegebene Position erreicht hat.

3. Verfahren nach einem der vorangehenden Ansprüche, wobei zumindest in dem Fall, dass sich die Nadelspitze (N) in einem Patienten befindet und/oder nach einer Bestimmung der Nadellänge (L), eine Messung der Position des Nadelhalters (31) erfolgt, insbesondere mittels eines kapazitiven Sensors, eines induktiven Sensors, eines optischen Sensors, oder eines akustischen Sensors oder aus dem Zustand eines Potentiometers oder einer Steuervorgabe, bevorzugt wobei eine Verschiebung des Nadelhalters (31) von einer Skala (S) abgelesen oder mittels einer Skala (S) ermittelt wird oder der Zustand eines Schiebeschlittens (36) der Biopsie-Einheit (30) ermittelt wird.

4. Verfahren nach einem der vorangehenden Ansprüche, wobei vor einer Biopsie und nach dem Positionieren der Biopsienadel (32) im Nadelhalter (31) eine Messung der Länge (L) der Biopsienadel (32) erfolgt, bevorzugt durch Aufnahme der Biopsienadel (32) im Nadelhalter (31) in einem Bild (B) und Auswertung des Bildes (B) oder durch Bestimmung einer Positionierung der Nadelspitze (N) an der Nadelführung (33) und anschließendem Verfahren des Nadelhalters (31) in Richtung der Nadelführung (33) bis zu einem Anschlagspunkt.

5. Verfahren nach Anspruch 4, wobei das Ermitteln der Position der Nadelspitze (N) der Biopsienadel (32) mittels einem optischen Sensor (35), bevorzugt einer Kamera erfolgt,
bevorzugt wobei ein Bild (B) der Biopsienadel (32) aus einer festgelegten Aufnahmeposition mit einem vorgegebenen Abstand zur Biopsienadel (32) aufgenommen wird und eine Länge (L) der Biopsienadel (32) berechnet wird, indem in dem Bild (B) die Biopsienadel (32) und der Nadelhalter (31) gesucht werden und die Länge (L) der Biopsienadel (32) im Bild (B) mittels einer vorgegebenen Funktion auf die wahre Länge (L) der Biopsienadel (32) umgerechnet wird und bevorzugt auch die Dicke der Biopsienadel (32) im Bild (B) mittels einer vorgegebenen Funktion auf die wahre Dicke der Biopsienadel (32) umgerechnet wird.

6. Verfahren nach einem der vorangehenden Ansprüche, wobei das Vorhandensein einer Biopsienadel (32) im Nadelhalter (31) mittels eines Sensors ermittelt wird und eine Verschiebung des Nadelhalters (31) im Falle, dass eine Biopsienadel (32) eingesetzt ist, bei vorgegebenen Situationen blockiert wird, insbesondere wenn ein Abstand von der Nadelspitze (N) zu einem festen Bezugspunkt einen vorgegebenen Grenzwert überschreitet.

7. Verfahren nach Anspruch 5 oder 6, wobei nach dem Positionieren der Biopsienadel (32) im Nadelhalter (31) und bevorzugt auch nach einer optionalen Überprüfung, ob eine Biopsienadel (32) im Nadelhalter (31) eingesetzt ist, die folgenden Schritte durchlaufen werden:
- Aufnehmen eines Bildes (B) von der Biopsienadel (32) und dem Nadelhalter (31),
- Segmentieren des Bildes (B), bevorzugt mittels eines maschinenlernfähigen Modells trainiert zur Erkennung von Biopsienadeln (32) und Nadelhaltern (31) in einem Bild (B),
- Durchführen einer Konturdetektion zumindest der Biopsienadel (32), bevorzugt mittels einer Minimalrechteck-Methode
- Zählen derjenigen Pixel, die als Biopsienadel (32) segmentiert worden sind, bevorzugt zumindest in vertikaler Richtung zur Ermittlung der Länge (L) der Biopsienadel (32) im Bild (B), vorzugsweise zusätzlich in horizontaler Richtung zur Ermittlung der Dicke der Biopsienadel (32) im Bild (B),
- Berechnen der Länge (L) der realen Biopsienadel (32) mittels der Länge (L) der Biopsienadel (32) im Bild (B) und einer vorgegebenen Formel und bevorzugt auch der Dicke der realen Biopsienadel (32) mittels der Dicke der Biopsienadel (32) im Bild (B) und einer vorgegebenen Formel,
- Ausgeben der berechneten Werte bevorzugt in Form eines Bildes (B) mit entsprechenden Bemaßungen.

8. Verfahren nach einem der Ansprüche 5 bis 7, wobei zusätzlich der Nadelhalter (31) nach Merkmalen untersucht wird, die dessen Hersteller offenbaren, insbesondere wobei im Bild (B) an der Position des Nadelhalters (31) nach Zeichen, insbesondere in Form von Logos und/oder Schriftzeichen, gesucht wird, und gefundene Zeichen mit einer Liste aus Zeichen verglichen werden.

9. Verfahren nach einem der vorangehenden Ansprüche, wobei vor Einsetzen der Biopsienadel (32) in den Nadelhalter (31) ein Identifikationscode, bevorzugt ein QR-Code, ein Label, eine Zeichenkette, eine NFC-Information oder ein Barcode, an der Biopsienadel (32) oder einer Verpackung der Biopsienadel (32) gescannt wird, die gescannte Information mit einer vorgegebenen Information über einen gewünschten Nadeltyp verglichen wird und in dem Fall dass der Barcode (C) anzeigt, dass es sich nicht um den gewünschten Nadeltyp handelt, eine Warninformation ausgegeben wird und/oder ein Einsetzen der Biopsienadel in den Nadelhalter (31) blockiert wird.

10. Vorrichtung (10) zur automatisierten Bestimmung der Position einer Nadelspitze (N) einer Biopsienadel (32), welche in einem Nadelhalter (31) angebracht ist und für eine Biopsie durch eine Biopsie-Einheit (30) auf ihrer longitudinalen Achse verschiebbar ist und mittels einer Nadelführung (33) geführt wird, die Vorrichtung (10) umfassend:
- optional: einen Sensor zum Erkennen eines Positionierens einer Biopsienadel (32) im Nadelhalter (31),
- eine Ermittlungseinheit (11), ausgelegt zum Ermitteln zumindest der Position der Nadelspitze (N) der Biopsienadel (32) mittels eines Sensors (35),
- eine Berechnungseinheit (12), ausgelegt zum automatisches Berechnen eines Abstandes von der Nadelspitze (N) zu einem Bezugspunkt.

11. Vorrichtung (10) nach Anspruch 10 umfassend einen optischen Sensor (35) ausgelegt und angeordnet zur Aufnahme von Bildern (B) des Nadelhalters (31) und einer eingelegten Biopsienadel (32), und umfassend eine Längenermittlungseinheit (13) ausgelegt zum Ermitteln der Länge (L) der Biopsienadel (32), bevorzugt indem in dem Bild (B) die Biopsienadel (32) und der Nadelhalter (31) gesucht werden und die Länge (L) der Biopsienadel (32) im Bild (B) mittels einer vorgegebenen Funktion auf die wahre Länge (L) der Biopsienadel (32) umgerechnet wird, und bevorzugt auch die Dicke der Biopsienadel (32) im Bild (B) mittels einer vorgegebenen Funktion auf die wahre Dicke der Biopsienadel (32) umgerechnet wird.

12. Vorrichtung (10) nach Anspruch 10 oder 11 umfassend einen Sensor (35) ausgelegt zur Detektion der Nadelspitze (N) an der Nadelführung (33) und einen Sensor (34) ausgelegt zur Bestimmung der Position des Nadelhalters (31), bevorzugt wobei die Berechnungseinheit (12) dazu ausgelegt ist, basierend auf der gemessenen Position der Nadelspitze (N) an der Nadelführung (33) und der Position des Nadelhalters (31) automatisch einen Abstand von der Nadelspitze (N) zu einem festen Bezugspunkt im Raum zu berechnen, bevorzugt zu einem Punkt am Nadelhalter (31).

13. Mammographiesystem (1) umfassend eine Vorrichtung (10) nach einem der Ansprüche 10 bis 12 und/oder ausgelegt zum Durchführen eines Verfahrens nach einem der Ansprüche 1 bis 9.

14. Computerprogrammprodukt, umfassend Befehle, die bei der Ausführung des Programms durch einen Computer diesen veranlassen, die Schritte des Verfahrens nach einem der Ansprüche 1 bis 9 auszuführen.

15. Computerlesbares Speichermedium, umfassend Befehle, die bei der Ausführung durch einen Computer diesen veranlassen, die Schritte des Verfahrens nach einem der Ansprüche 1 bis 9 auszuführen.
